(19) Europäisches Patentamt
European Patent Office
Office européen des brevets

(11) EP 0 465 344 B1

(12) EUROPEAN PATENT SPECIFICATION

(45) Date of publication and mention of the grant of the patent:
**04.09.1996 Bulletin 1996/36**

(51) Int Cl.[6]: **C09K 19/32**

(21) Application number: **91401807.2**

(22) Date of filing: **02.07.1991**

(54) **Liquid crystal compounds**

Flüssigkristallinverbindungen

Composés à cristaux liquides

(84) Designated Contracting States:
**DE FR GB**

(30) Priority: **05.07.1990 JP 178336/90**

(43) Date of publication of application:
**08.01.1992 Bulletin 1992/02**

(73) Proprietor: **Showa Shell Sekiyu Kabushiki Kaisha
Tokyo (JP)**

(72) Inventors:
- **Aihara, Yoshihiko, c/o Showa Shell Sekiyu K.K. Chiyoda-ku, Tokyo 100 (JP)**
- **Isozaki, Tadaaki, c/o Showa Shell Sekiyu K.K. Chiyoda-ku, Tokyo 100 (JP)**
- **Ooide, Toru, c/o Showa Shell Sekiyu K.K. Chiyoda-ku, Tokyo 100 (JP)**
- **Yamakawa, Noriko, c/o Showa Shell Sekiyu K.K. Chiyoda-ku, Tokyo 100 (JP)**

(74) Representative: **Bourgognon, Jean-Marie et al
Cabinet Flechner
22, Avenue de Friedland
75008 Paris (FR)**

(56) References cited:
**EP-A- 0 339 987** **EP-A- 0 341 922**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 465 344 B1

## Description

The present invention is to provide an antiferroelectric liquid crystal compound having a naphthalene skeleton, and the liquid crystal compound relates to liquid crystal compounds used for display devices or electrooptical devices which take advantage of the response to electric field.

Furthermore, the present invention relates to an antiferroelectric liquid crystal compound exhibiting tristable molecular alignments. The liquid crystal compound is used for display devices or electrooptical devices which take advantage of the response to electric field.

As electrooptical devices using nematic liquid crystals such as those of DSM, TN, G-H or STN types have been developed and put to practical use. These electrooptical devices using the nematic liquid crystals have a defect of a very slow response in the range from several milliseconds to several ten milliseconds and thus are limited in their applications. The slow response of devices utilizing a nematic liquid crystal is attributed to the fact that the torque for moving molecules is based on the anisotropy of dielectric constant and thus the power is not strong. Among these backgrounds, a ferroelectric liquid crystal which exhibits spontaneous polarization (Ps) and a strong torque based on Ps x E (E: applied electric field) and is capable of a high speed response in the range of several µsec to several ten µsec has been developed by Meyer et al. (Le Journal de Physique, 36, 1975, L-69). Furthermore, there is disclosed a new antiferroelectric liquid crystal in Japanese Patent Laid-Open Publication No. 307838/1988. There are also the disclosed "tristable states" described later in Japanese Patent Laid-Open Publication Nos. 316339/1989, 316367/1989, 316372/1989 and 28128/1990 by the present applicant.

EP-A-0339987 relates to a liquid crystal compounds of naphtalene nucleus having an optically active group, which exhibit tri-stable phases.

EP-A-0341922 discloses naphtalene compounds containing an optically active group and their use in a liquid crystal composition.

There have already been proposed several high speed electrooptical devices comprising ferroelectric liquid crystals.

A typical example includes a device in which the helical structure is released by the force of wall faces and the two molecular alignments parallel to the wall faces is changed by the polarity of an applied electric field (see, for example, Japanese Patent Laid-Open Publication No. 107216/1981).

The aforementioned device is composed on the assumption of the presence of a compound which exhibits such an ideal bistable states as is shown by a field response wave pattern in Fig.1. However, no compound which exhibits such an ideal bistable states as described above has not been found, and bistable liquid crystals synthesized hitherto show a field response wave pattern in Fig.2 but not a field response wave pattern in Fig.1. It is the present state that if a device which exhibits a response wave pattern as shown in Fig.2 is intended to be used for a switching circuit of light, such a pattern has a profile that transmittance varies gradually with the variation of an applied voltage from the minus side to the plus side and thus the object cannot be accomplished sufficiently with such a simple change of applied voltage as "on" and "off". Moreover, a bistable state liquid crystal having been synthesized is hard to form a monodomain structure as an ideal molecular alignment in the stage of a S*c phase at no electric field, and it causes disclination (defect) or twist which is the disturbance of the molecular alignment. It is thus difficult to realize the aforementioned ideal bistable alignment in a large area. Furthermore, it has a low threshold value (voltage at which the brightness varies at a predetermined extent), so that the dynamic drive of it may cause the lowering of contrast or the decrease of the range of viewing angle. The bistable state liquid crystal hitherto synthesized has no memory effect, since it cannot exhibit a hysteresis as shown in Fig. 1 but exhibits only a hysteresis as shown in Fig. 2. Thus, it is necessary to impress continuously a voltage at $v_3$ in Fig. 2 or to apply a high frequency in order to maintain a stable response in the S*c phase in the liquid crystal, and it cannot avoid a large energy loss.

Eventually, it is the present state that many problems remain unsolved in conventional ferroelectric liquid crystal electrooptical devices notwithstanding the earnest desire of a high speed liquid crystal electrooptical device which takes advantage effectively of an applied field and a bond having a strong molecular alignment obtained in a liquid crystal.

Thus, the object of the present invention is to provide a novel liquid crystal compound which can be used in the liquid crystal electrooptical device described in Japanese Patent Laid-Open Publication No. 153322/1990 and takes advantage of the tristable states, characterized in that the liquid crystal compound realizes a stable molecular alignment having a distinct light-dark contrast depending on applied electric field, generates a distinct threshold property and a distinct hysteresis as shown in Fig. 3, realizes easily dynamic drive and is capable of a high speed response.

Fig. 1 shows the hysteresis of an ideal bistable state liquid crystal which has not in fact been obtained,

Fig. 2 shows the hysteresis of a practical bistable state liquid crystal having been hitherto synthesized,

Fig. 3 shows the hysteresis of tristable state liquid crystal according to the present invention, respectively, in which the abscissa represents applied voltage and the ordinate represents transmittance (%),

Fig. 4-A represents a triangular wave applied, and Figs. 4-B, 4-C and 4-D represent the optical response properties of a commercially available nematic liquid crystal, a bistable state liquid crystal and a tristable state liquid crystal, respectively, and

Figs. 5 and 6 represent the infrared spectra of the compounds according to the present invention in Examples 1 and 2.

The object of the present invention consists in providing a novel ferroelectric liquid crystal having quite new tristable states which is distinguished from a ferroelectric liquid crystal exhibiting a chiral smectic phase or a chiral smectic C phase (S*c phase) as the conventional bistable states.

The term "having tristable states" means that in a liquid crystal electrooptical device comprising an antiferroelectric liquid crystal inserted between the first electrode plate and the second electrode plate arranged at a predetermined distance from the first electrode plate, characterized in that the device is constituted so that the voltage for forming electric field is applied to the first and second electrode plates, and, on applying the voltage in the shape of a triangular wave as shown in Fig. 4-A,the antiferroelectric liquid crystal has the first stable state of the molecular alignment at no electric field (at 2 in Fig. 4-D), the second stable state different from the first stable state for the one direction of the electric field on applying the electric field (at 1 in Fig. 4-D),and the third stable state different from the first and second stable states for the other direction of the electric field (at 3 in Fig. 4-D). In this connection, the liquid crystal electrooptical device taking advantage of the tristable states has been filed as Japanese Patent Application No. 70212/1988 by the present applicants.

On the other hand, "the commercially available nematic liquid crystal" or the bistable state liquid crystals having been synthesized have not tristable states but the optical response shown in Figs. 4-B or C.

The novel tristable state antiferroelectric liquid crystal exhibits an epoch-making effect as a liquid crystal display in comparison with the conventional nematic liquid crystals.

While the conventional liquid crystal having a high image quality is required to take a very complicated structure of the driving mode such as the active matrix mode, the tristable state antiferroelectric liquid crystal is required only a simple display of a matrix type. The conventional type was produced by a complicated process, so that an image plane was difficult to be made in a large size and the production cost was expensive. On the other hand, in the case of the tristable antiferroelectric liquid crystal the production process is simple and epoch-making, so that it is possible to make the image plane in a large size and to control the production cost at an inexpensive level.

The object of the present invention is to provide a novel liquid crystal having the tristable antiferroelectricity.

The present invention is defined in claim 1 and improvements of it in subsequent claims 2 to 5.

The two synthetic methods of the compound according to the present invention will be described with reference to examples.

[A] 4-Hydroxybiphenyl-4'-carboxylic acid was dissolved in methanol and heated under refluxing in the presence of concentrated sulfuric acid to give 4-hydroxy-biphenyl-4'-carboxylic acid methyl ester. Then the ester and benzyl chloride were dissolved in a solvent such as DMF and were allowed to react in the presence of anhydrous potassium carbonate to give 4-benzyloxy-biphenyl-4'-carboxylic acid. The compound was further allowed to react with thionyl chloride to give 4-benzyloxy-biphenyl-4'-carboxylic acid chloride.

An optically active 1,1,1-trifluoro-2-alkanol and the aforementioned acid chloride were allowed to react in a solvent such as methylene chloride to give 1-trifluoromethylalkyl 4-benzyloxy-4'-biphenyloarboxylate. The ester was subjected to hydrogenolysis to give 1,1,1-trifluoro-2-alkyl 4'-hydroxybiphenyl-4-carboxylate.

An alkyl bromide and 2-hydroxynaphthalene-6-carboxylic acid methyl ester were allowed to react in the presence of potassium carbonate in a solvent such as dimethylformamide and then subjected to hydrolysis with an aqueous sodium hydroxide solution. The hydrolyzed product was allowed to react with the 1,1,1-trifluoro-2-alkyl 4'-hydroxybiphenyl-4-carboxylate in the presence of dicyclohexylcarbodiimide to give 6-alkyloxynaphthalene-2-carboxylic acid 4'-(1,1,1-trifluoro-2-alkyloxycarbonyl)biphenyl-4-ester.

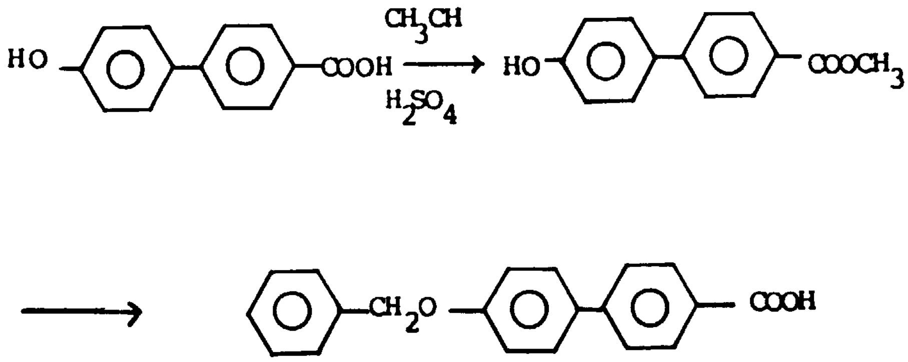
$CH_3CH$
$H_2SO_4$

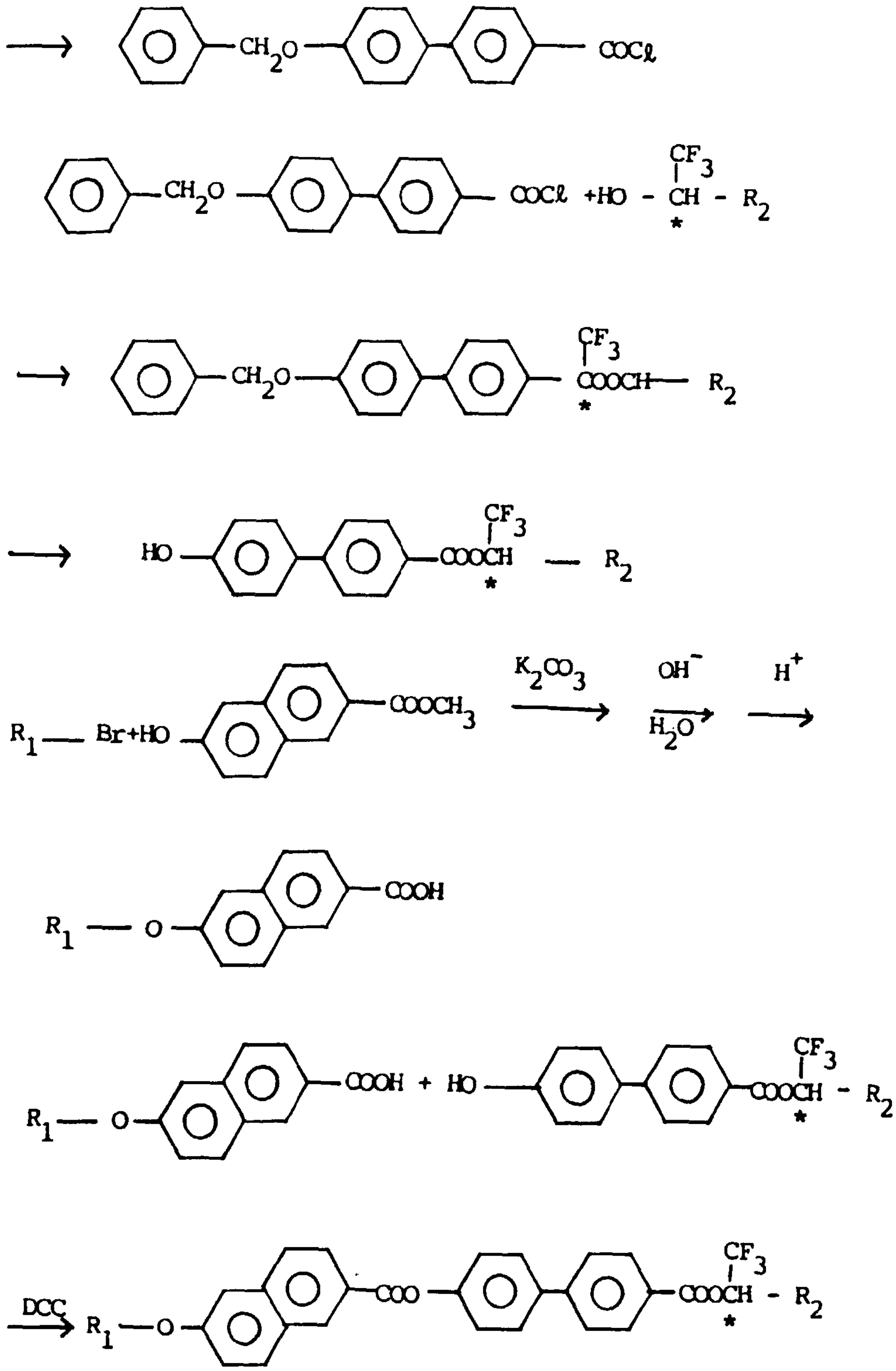


[B] 4,4'-Biphenyldicarboxylic acid chloride and an optically active 1,1,1-trifluoro-2-alkanol were allowed to react to give 4'-(1,1,1-trifluoro-2-alkyloxycarbonyl)biphenyl-4-carboxylic acid chloride.

An alkyl bromide and 2-hydroxy-6-methylcarbonyloxynaphthalene were allowed to react in the presence of potassium carbonate in a solvent such as dimethylformamide and then subjected to hydrolysis with an aqueous sodium hydroxide solution. The hydrolyzed product was allowed to react with the aforementioned 4'-(1,1,1-trifluoro-2-alkyloxycarbonyl)biphenyl-4-carboxylic acid chloride to give 6-alkyloxynaphthalene 4-(1,1,1-trifluoro-2-alkyloxycarbonyl)biphenyl-4'-carboxylate.

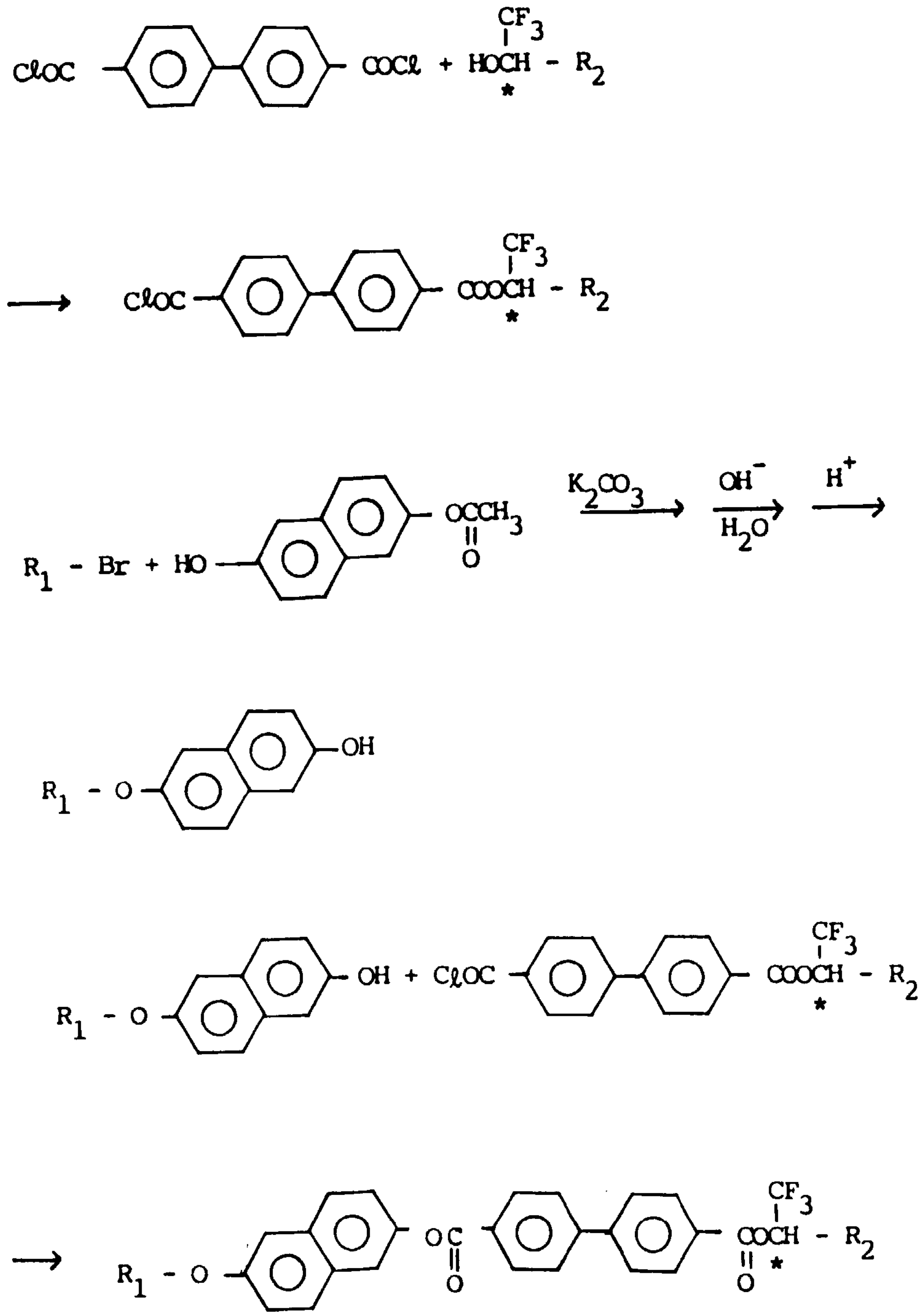


The compounds of the present invention are described with reference to examples without limitation thereto.

Example 1

(1) Synthesis of 4'-(1,1,1-trifluoro-2-octyloxycarbonyl)biphenyl-4-carboxylic acid chloride

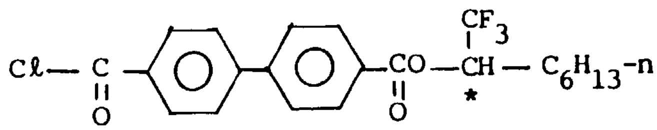

An optically active 1,1,1-trifluoro-2-octanol (1.8 g) and triethylamine (1.1 g) were dissolved in 80 ml of methylene chloride. To this solution was added slowly 4,4'-biphenyldicarboxylic acid chloride (2.3 g). Dimethylaminopyridine (0.3 g) was further added, and the mixture was stirred for a day. The solvent was removed by distillation to give 4 g of residue containing 4-(1,1,1-trifluoro-2-octyloxycarbonyl)biphenyl-4'-carboxylic acid chloride.

(2) Synthesis of 2-n-decyloxy-6-hydroxynaphthalene

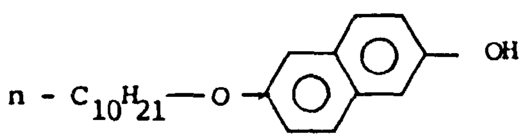

Decyl monobromide (7 g), 2-hydroxy-6-methylcarbonyloxynaphthalene (2 g) and anhydrous potassium carbonate (4 g) were added to 100 ml of dimethylformamide. After stirring at 130°C for 2 hours, the reaction solution was poured into water. Dilute hydrochloric acid was added to neutralize the solution, which was extracted with diethyl ether. The residue obtained from the diethyl ether layer by removing the solvent by distillation was added to a solution comprising 3.3 g of sodium hydroxide, 10 ml of water and 50 ml of ethanol, and the mixture was heated under refluxing for a day. Dilute hydrochloric acid was added to neutralize the solution, which was concentrated by removing the solvent by distillation. Solid products deposited were collected and recrystallized from a mixed solvent of water-ethanol to give 2.4 g of the desired product.

(3) Synthesis of 6-n-decyloxynaphthalene 4'-(1,1,1-trifluoro-2-octyloxycarbonyl)biphenyl-4- carboxylate

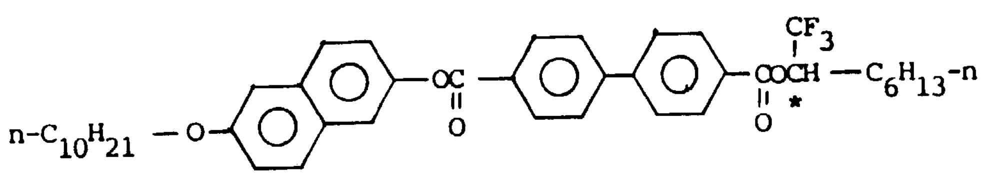

2-n-Decyloxy-6-hydroxynaphthalene (0.5 g) obtained in (2) of Example 3 and triethylamine (0.25 g) were dissolved in 50 ml of methylene chloride. The residue (about 4 g) containing 4'-(1,1,1-trifluoro-2-octyloxycarbonyl)biphenyl-4-carboxylic acid chloride obtained in (1) of Example 3 was added gradually to the solution above. Dimethylaminopyridine (0.02 g) was further added, and the mixture was stirred for a day. The solution was washed with dilute sulfuric acid and water in this sequence, and the organic layer was collected and dried over anhydrous magnesium sulfate. The solvent was removed by distillation, and the residue was purified by column chromatography on silica gel (eluent: hexane/ethyl acetate = 20/1) to obtain 0.46 g of the desired product.

The product had a specific rotation of $[\alpha]_D^{20}$ = +37.67° and the following phase transition temperatures were observed under a microscope equipped with a hot stage:

Cry ← Sx ← S*(3) ← S*c ← SA ← Iso

0 10 75 106 150.5

Sx is a phase of a higher order having a response to electric field. The infrared spectrum (KBr) of the desired product is shown in Fig. 5.

Example 2

(1) Synthesis of 6-n-decyloxynaphthalene-2-carboxylic acid 4-(1,1,1-trifluoro-2-octyloxycarbonyl)biphenyl-4'-ester

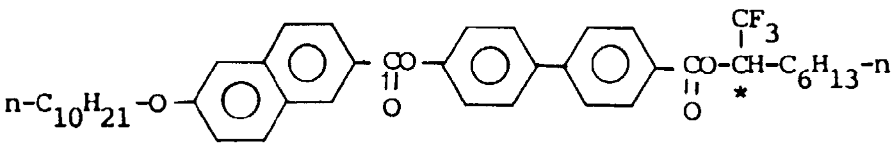

2-n-Decyloxy-5-carboxynaphthalene (0.6 g) obtained in (3) of Example 1 and optically active 1,1,1-trifluoro-2-octyl 4'-hydroxybiphenyl-4-carboxylate (0.65 g) obtained in (1) of Example 2 were dissolved in 50 ml of tetrahydrofuran. Dicyclohexylcarbodiimide (0.6 g) and dimethylaminopyridine (0.1 g) were added to the solution above, and the mixture was stirred at room temperature for a day. After removing the solvent by distillation, the residue was dissolved in 50 ml of dichloromethane. The solution was washed with dilute sulfuric acid and water in this sequence, and the organic layer was collected and dried over anhydrous magnesium sulfate. The solvent was removed by distillation, and the residue was purified by chromatography on silica gel (eluent: hexane/ethyl acetate = 20/1) to give 0.6 g of the desired product.

The product had a specific rotation of $[\alpha]_D^{20}$ = +35.52° and the following phase transition temperatures were observed under a microscope equipped with a hot stage:

$$\mathrm{Cry} \xleftarrow{35.4} \mathrm{S^*(3)} \xleftarrow{97} \mathrm{S^*C} \xleftarrow{120} \mathrm{SA} \xleftarrow{151.9} \mathrm{Iso}$$

The infrared spectrum (KBr) of the desired product is shown in Fig. 6.

All of the novel liquid crystals of the present invention exhibit tristable states when in S* (3) phase and have a wide range of applications such as a display device or a switching device using the tristable states.

## Claims

**1.** A liquid crystal compound represented by the formula (I):

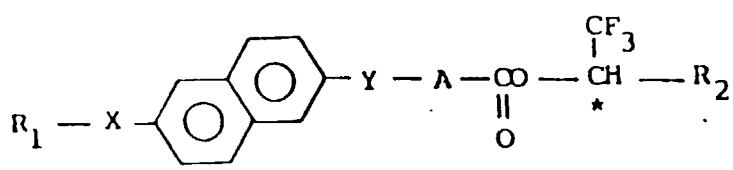 (I)

wherein

$R_1$ represents an alkyl group having 5 - 18 carbon atoms,
$R_2$ represents an alkyl group having 6 - 16 carbon atoms,
Y represents a group

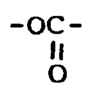 or 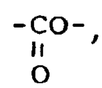,

A represents a group

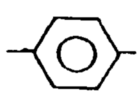 or 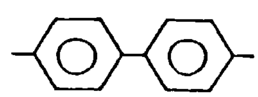,

and X represents

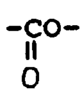 or 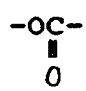

when A is a group

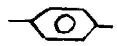

or X represents -O- or

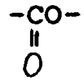

when A is a group

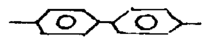

2. A liquid crystal compound according to claim 1 which is able to exhibit optically tristable states.

3. A liquid crystal compound which is able to exhibit optically tristable states and has the formula (II):

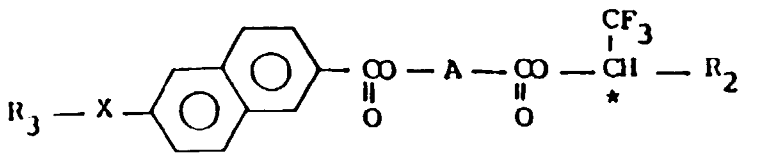
(II)

wherein $R_2$ represents an alkyl group having 6-16 carbon atoms, $R_3$ represents an alkyl group having 10-16 carbon atoms,
A represents a group

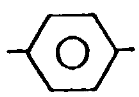 or 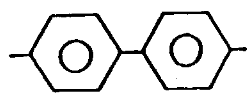

and X represents

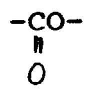 or 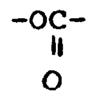

when A is a group

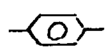,

or X represents -O- or

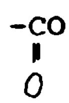

when A is a group

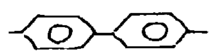.

4. A liquid crystal compound which is able to exhibit optically tristable states and has the formula (IV):

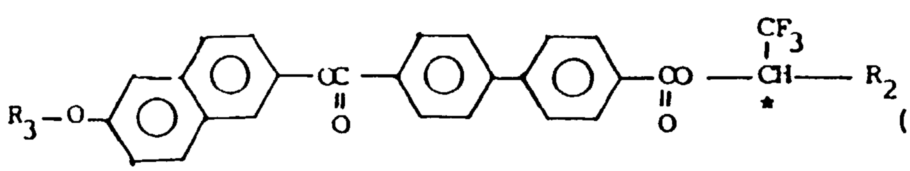
(IV)

wherein $R_2$ and $R_3$ are defined in claim 3.

5. A liquid crystal compound which is able to exhibit optically tristable states and has the formula (VI):

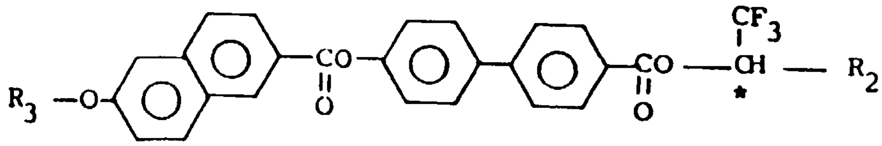

(VI)

wherein $R_2$ and $R_3$ are defined in claim 3.

**Patentansprüche**

**1.** Flüssigkristall-Verbindung, wiedergegeben durch die Formel (I):

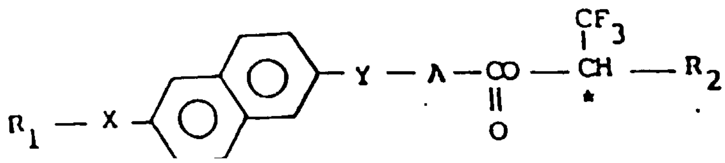

(I)

worin

$R_1$ für eine Alkylgruppe mit 5 bis 18 Kohlenstoffatomen steht;
$R_2$ für eine Alkylgruppe mit 6 bis 16 Kohlenstoffatomen steht;
Y für eine Gruppe

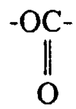 oder 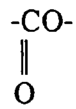

steht;
A für eine Gruppe

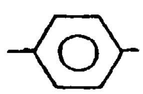 oder 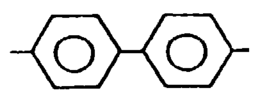

steht; und
X für

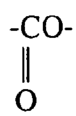 oder 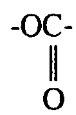

steht, wenn A für die Gruppe

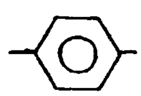

steht, oder
X für -O- oder

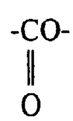

steht, wenn A für eine Gruppe

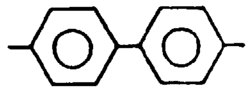

steht.

2. Flüssigkristall-Verbindung nach Anspruch 1, die in der Lage ist, optisch tristabile Zustände zu zeigen.

3. Flüssigkristall-Verbindung, die in der Lage ist, optisch tristabile Zustände zu zeigen, und die die Formel (II) aufweist:

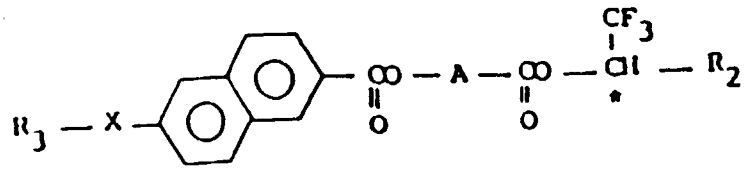

(II)

worin

$R_2$ für eine Alkylgruppe mit 6 bis 16 Kohlenstoffatomen steht;
$R_3$ für eine Alkylgruppe mit 10 bis 16 Kohlenstoffatomen steht;
A für eine Gruppe

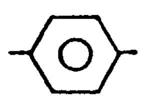 oder 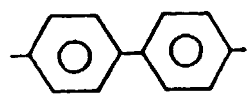

steht; und
X für

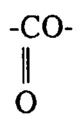 oder 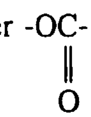

steht, wenn A für die Gruppe

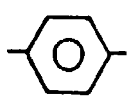

steht, oder
X für -O- oder

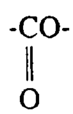

steht, wenn A für eine Gruppe

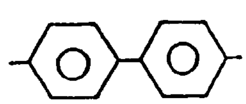

steht.

4. Flüssigkristall-Verbindung, die in der Lage ist, optisch tristabile Zustände zu zeigen, und die die Formel (IV) aufweist:

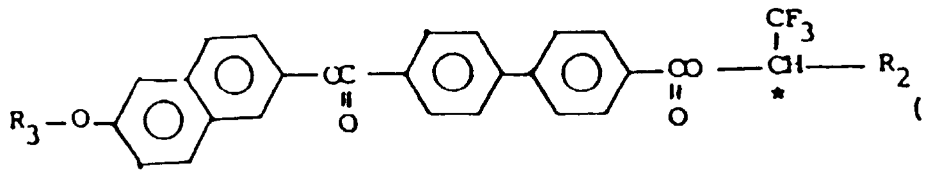

(IV)

worin $R_2$ und $R_3$ wie in Anspruch 3 definiert sind.

**5.** Flüssigkristall-Verbindung, die in der Lage ist, optisch tristabile Zustände zu zeigen, und die die Formel (VI) aufweist:

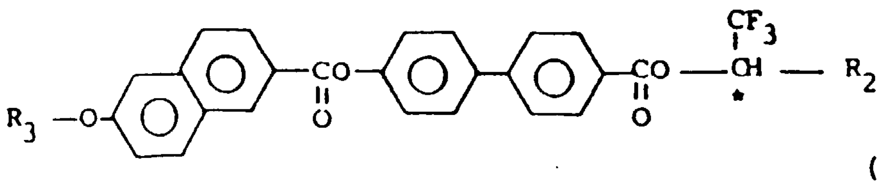

(VI)

worin $R_2$ und $R_3$ wie in Patentanspruch 3 definiert sind.

**Revendications**

**1.** Cristal liquide représenté par la formule (I) :

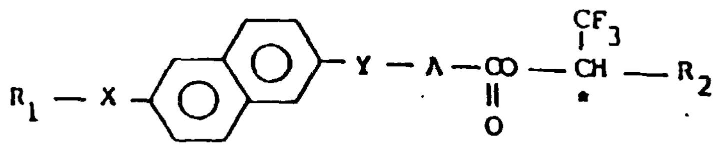

(I)

dans laquelle

$R_1$ représente un groupe alcoyle ayant de 5 à 18 atomes de carbone,
$R_2$ représente un groupe alcoyle ayant de 6 à 16 atomes de carbone,
Y représente un groupe

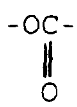 ou 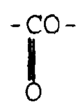

A représente un groupe

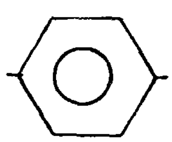 ou 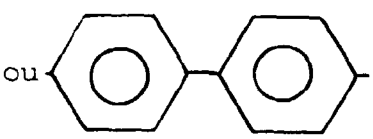

et X représente

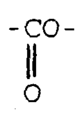 ou 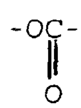

quand A est un groupe

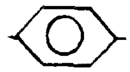

ou encore X représente -O- ou

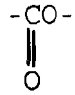

quand A est un groupe

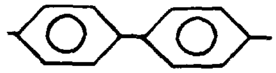

2. Cristal liquide selon la revendication 1, qui est à même de présenter des états optiquement tristables.

3. Cristal liquide à même de présenter des états optiquement tristables, et qui a la formule (II) :

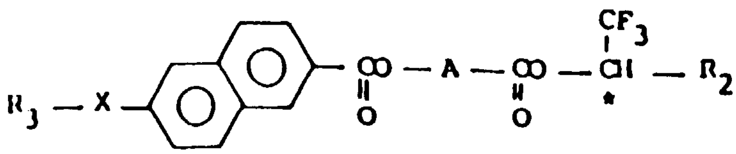
(II)

dans laquelle $R_2$ représente un groupe alcoyle ayant de 6 à 16 atomes de carbone, $R_3$ représente un groupe alcoyle avant de 10 à 16 atomes de carbone,

A représente un groupe

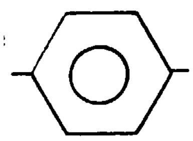 ou 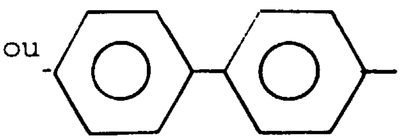

et X représente

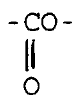 ou 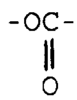

quand A est un groupe

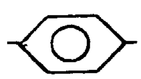

ou encore X représente -O- ou

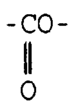

quand A est un groupe

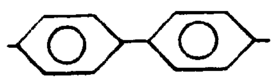

4. Cristal liquide, à même de présenter des états optiquement tristables, et ayant la formule (IV) :

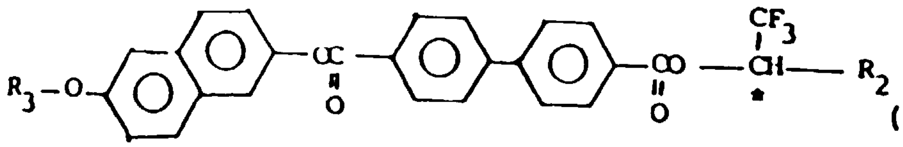

(IV)

dans laquelle $R_2$ et $R_3$ sont tels que définis dans la revendication 3.

**5.** Cristal liquide, à même de présenter des états optiquement tristables, et qui a la formule (VI) :

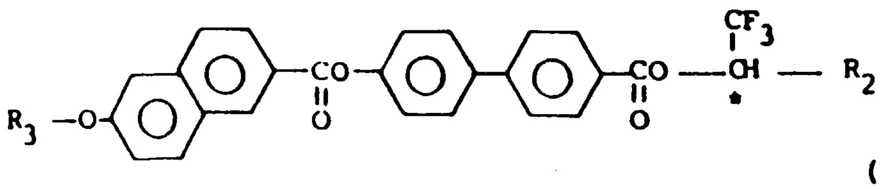

(VI)

dans laquelle $R_2$ et $R_3$ sont tels que définis dans la revendication 3.

FIG. 1

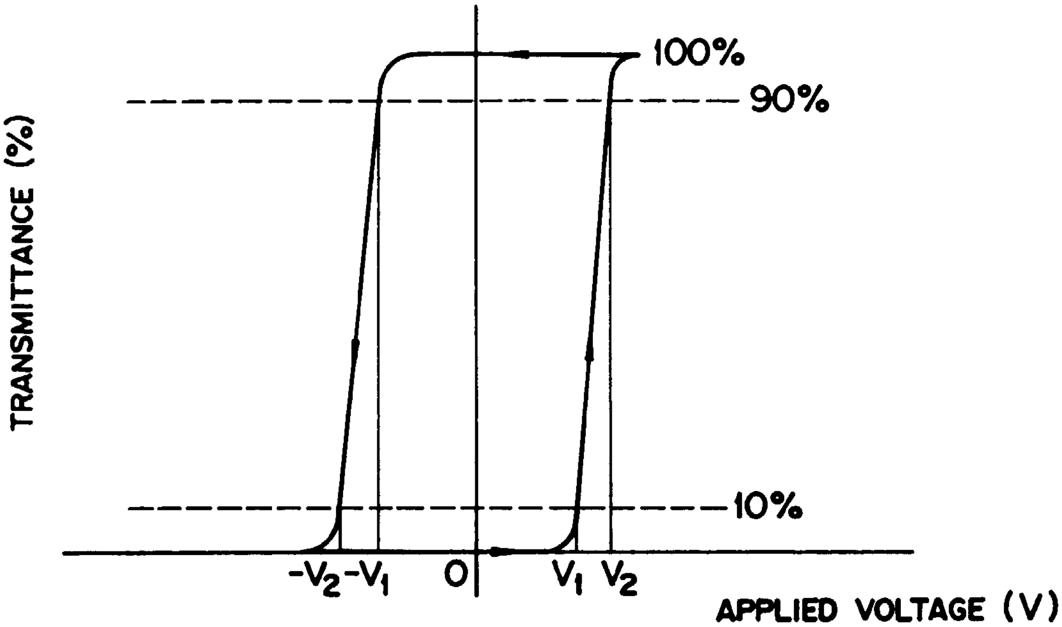
100%
90%
10%
TRANSMITTANCE (%)
-V2 -V1 0 V1 V2
APPLIED VOLTAGE (V)

FIG. 2

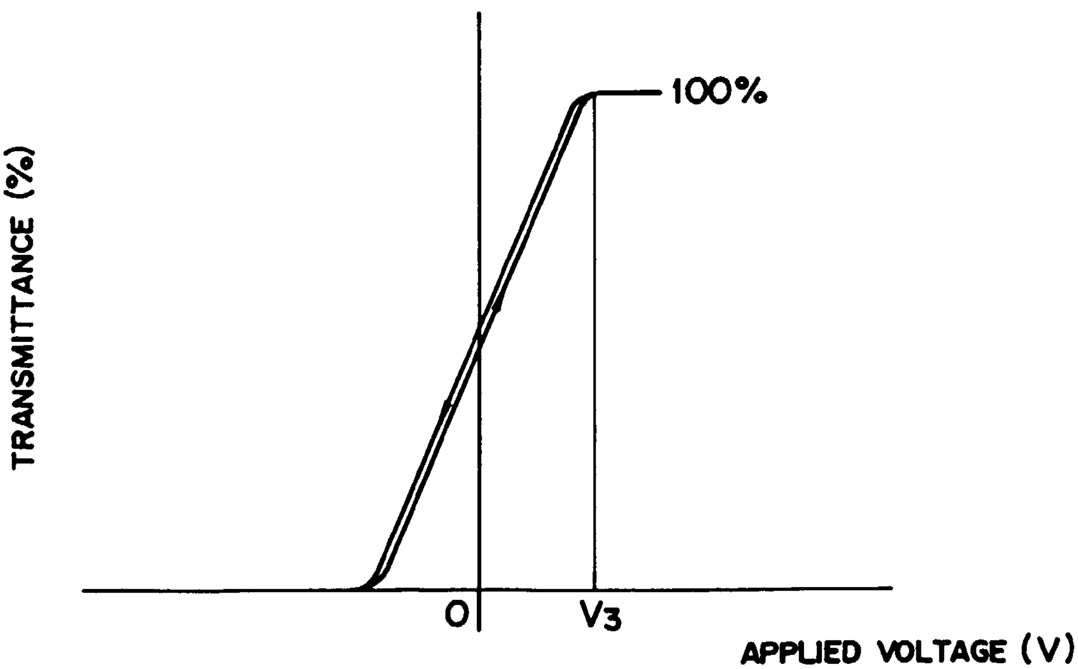
100%
TRANSMITTANCE (%)
0 V3
APPLIED VOLTAGE (V)

FIG. 3

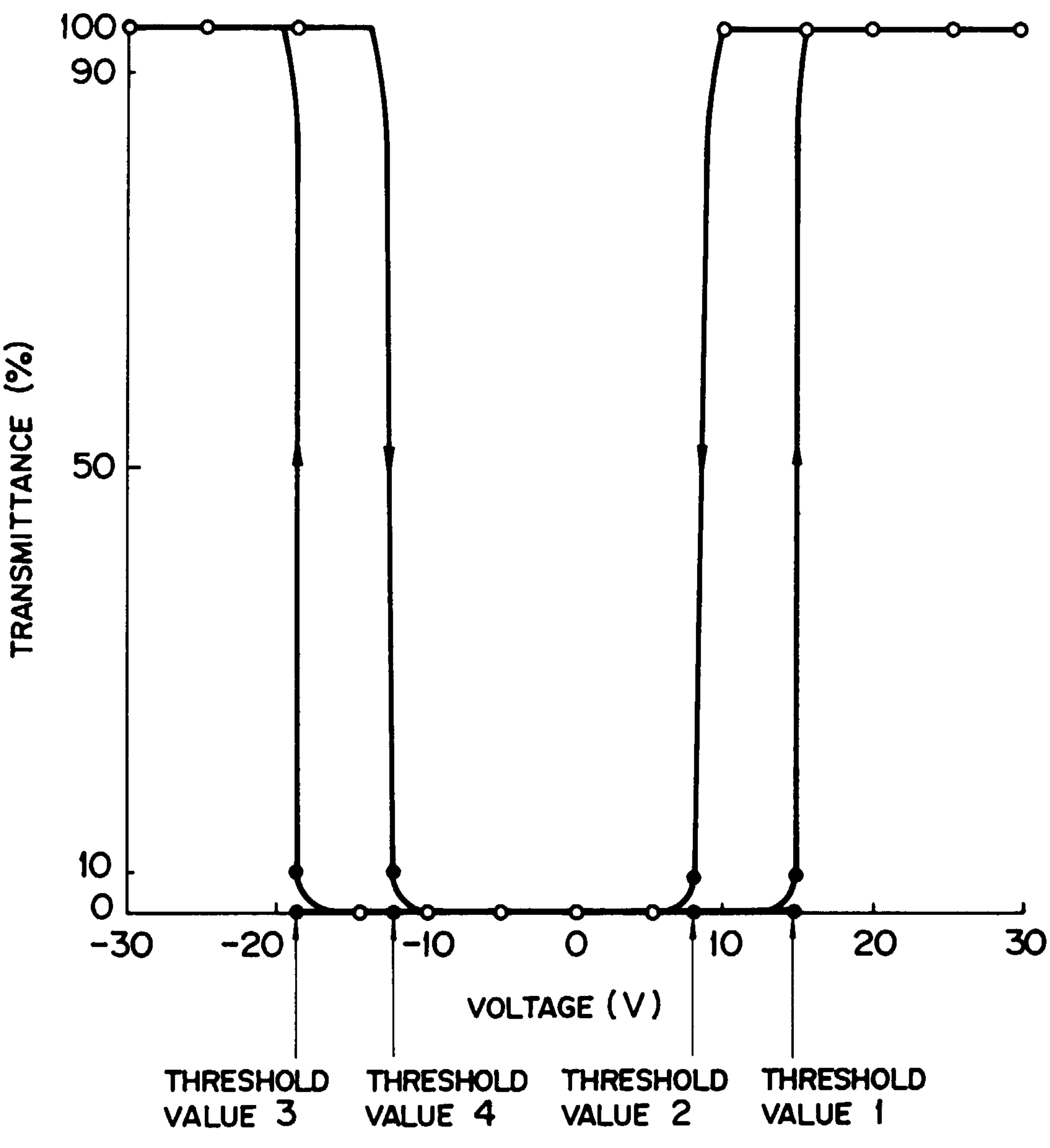
100
90
50
10
0
TRANSMITTANCE (%)
-30
-20
-10
0
10
20
30
VOLTAGE (V)
THRESHOLD VALUE 3
THRESHOLD VALUE 4
THRESHOLD VALUE 2
THRESHOLD VALUE 1

FIG. 4

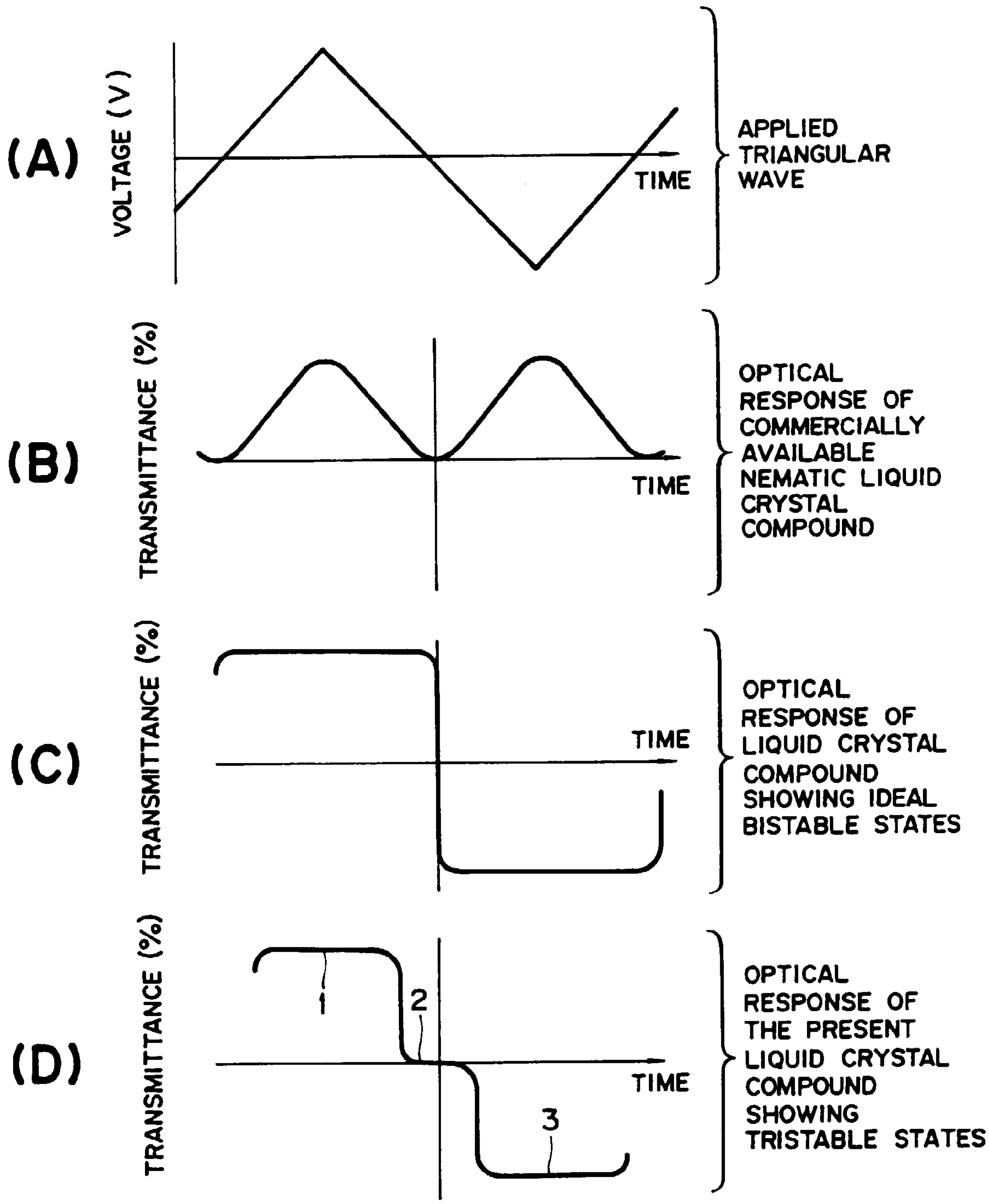
(A)
VOLTAGE (V)
TIME
APPLIED TRIANGULAR WAVE
(B)
TRANSMITTANCE (%)
TIME
OPTICAL RESPONSE OF COMMERCIALLY AVAILABLE NEMATIC LIQUID CRYSTAL COMPOUND
(C)
TRANSMITTANCE (%)
TIME
OPTICAL RESPONSE OF LIQUID CRYSTAL COMPOUND SHOWING IDEAL BISTABLE STATES
(D)
TRANSMITTANCE (%)
1
2
3
TIME
OPTICAL RESPONSE OF THE PRESENT LIQUID CRYSTAL COMPOUND SHOWING TRISTABLE STATES

FIG. 5

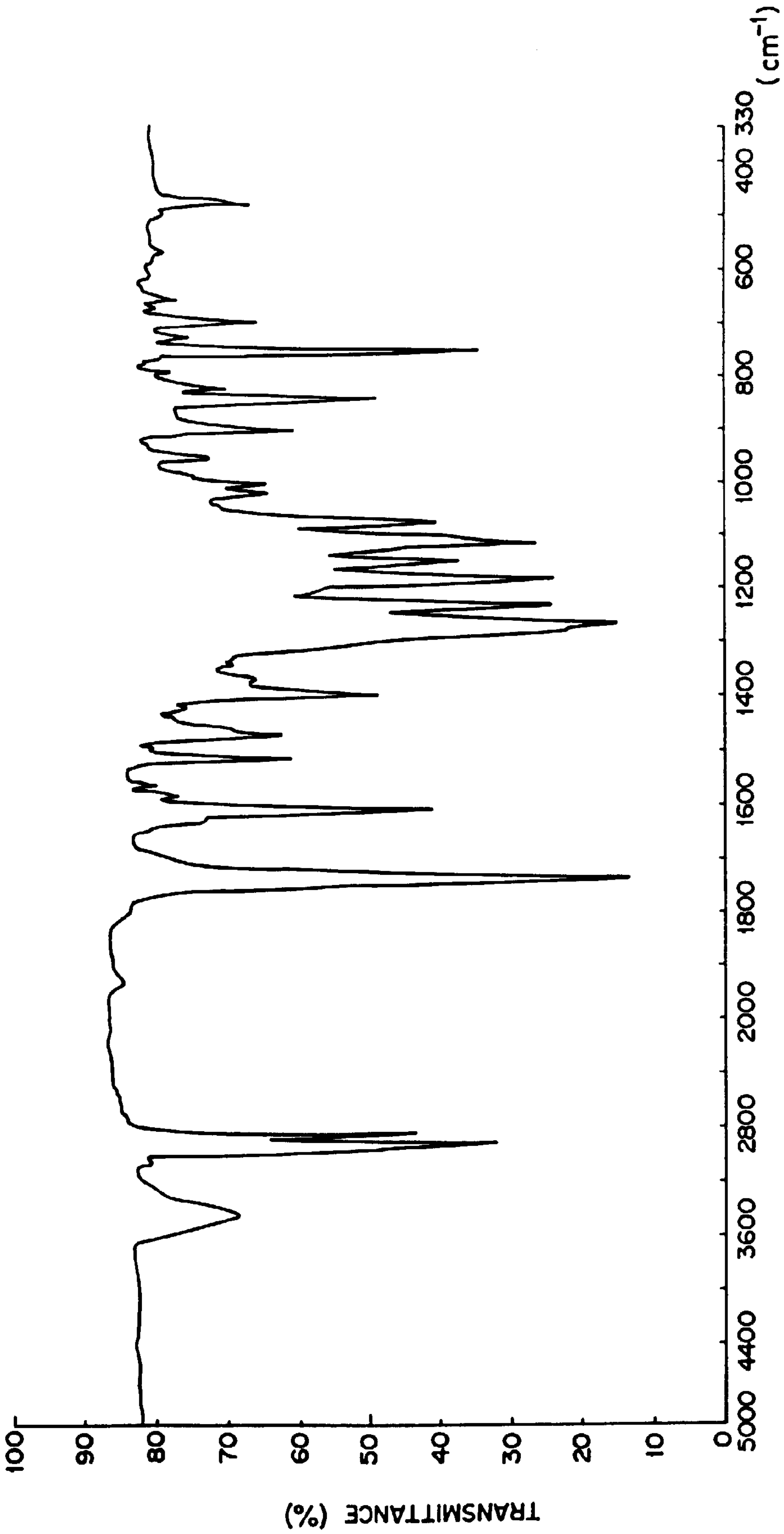
TRANSMITTANCE (%)
100
90
80
70
60
50
40
30
20
10
0
5000
4400
3600
2800
2000
1800
1600
1400
1200
1000
800
600
400
330
(cm$^{-1}$)
WAVE NUMBER

FIG. 6

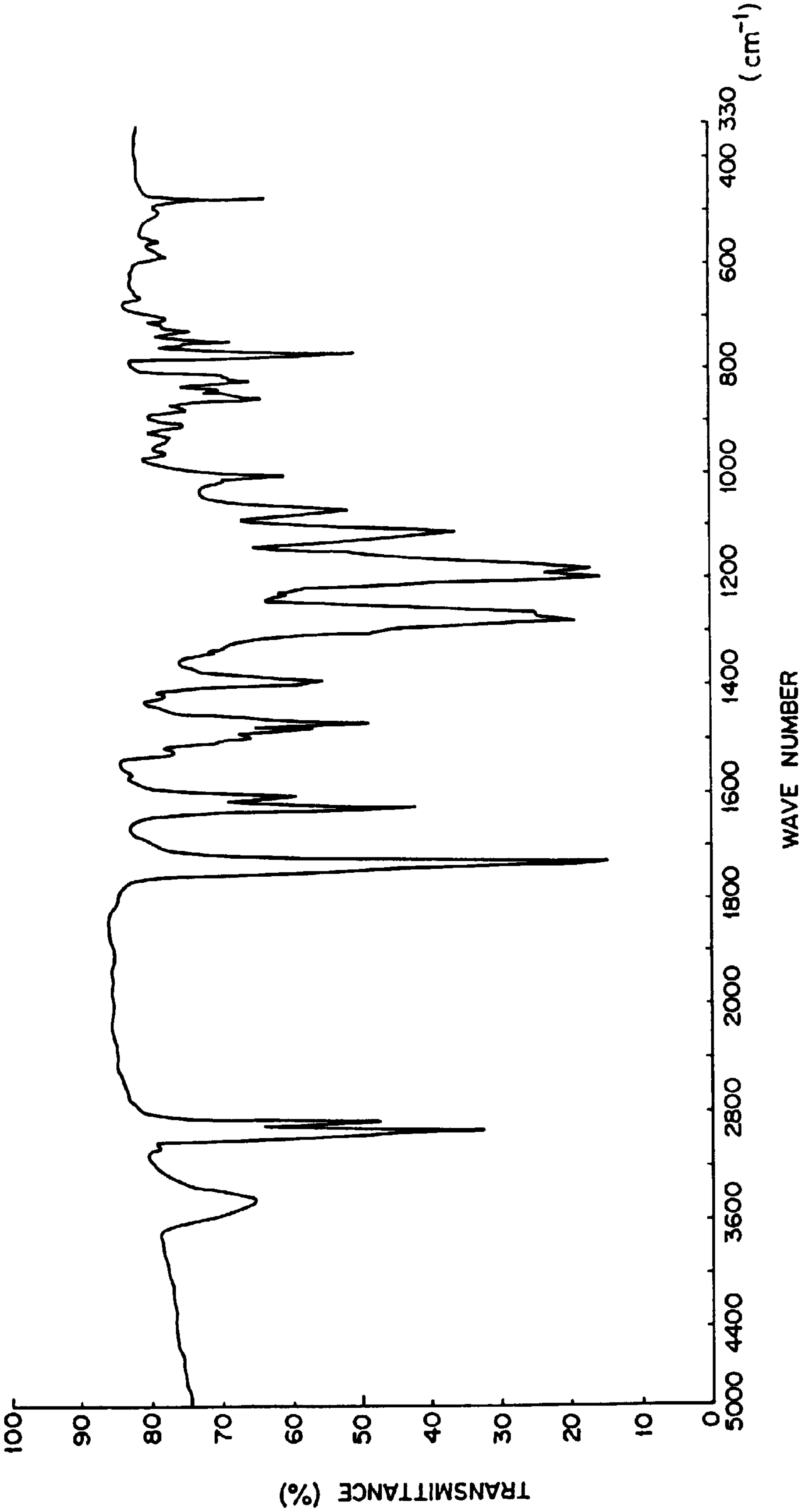
TRANSMITTANCE (%)
100
90
80
70
60
50
40
30
20
10
0
5000
4400
3600
2800
2000
1800
1600
1400
1200
1000
800
600
400
330
(cm$^{-1}$)
WAVE NUMBER